Europäisches Patentamt

European Patent Office

Office européen des brevets

(19)

(11) Numéro de publication: **0 016 293**
**B1**

(12) **FASCICULE DE BREVET EUROPEEN**

(45) Date de publication du fascicule du brevet: **27.06.84**

(21) Numéro de dépôt: **79400908.4**

(22) Date de dépôt: **23.11.79**

(51) Int. Cl.³: **A 61 F 5/00,** A 61 F 5/01, A 61 F 5/02

(54) Appareil orthopédique pour le traitement de la colonne vertébrale.

(30) Priorité: **23.03.79 FR 7907735**

(43) Date de publication de la demande:
**01.10.80 Bulletin 80/20**

(45) Mention de la délivrance du brevet:
**27.06.84 Bulletin 84/26**

(84) Etats contractants désignés:
**AT BE CH FR GB IT LU NL SE**

(56) Documents cités:
**CH - A - 505 616
FR - A - 1 037 220
FR - A - 1 276 078
FR - A - 2 031 200
FR - A - 2 290 884
FR - A - 2 349 321
FR - A - 2 426 453
GB - A - 740 507**

**Le dossier contient des informations techniques présentées postérieurement au dépôt de la demande et ne figurant pas dans le présent fascicule.**

(73) Titulaire: **Mignard, Jean Louis Laurent Lucien
75, Rue de la Plage
F-62600 Berck-Plage (FR)**

(72) Inventeur: **Mignard, Jean Louis Laurent Lucien
75, Rue de la Plage
F-62600 Berck-Plage (FR).**

(74) Mandataire: **Rinuy, Guy et al,
14, Avenue de la Grande Armée
F-75017 Paris (FR)**

Courier Press, Leamington Spa, England.

# Description

La présente invention concerne un appareil orthopédique permettant de corriger la déformation de la colonne vertébrale tel que scoliose, cyphose, hyperlordose, etc....

Jusqu'à présent, les patients présentant de telles déformations étaient munis de corsets qui sont soit d'une seule pièce pour maintenir le tronc dans une position donnée en enserrant la cage thoracique, soit constitués par des montants réglables sur lesquels sont fixées des ferrures transversales comportant de nombreux appuis rigides. Ces corsets, appliqués généralement sur les malades après traitement par un plâtre, ne permettent d'obtenir qu'une correction passive. Même si l'on y adjoint une minerve rigide ou un système de traction par sangle, on constate une régression de la correction obtenue grâce à l'emploi de plâtres. D'autre part, ils présentent un encombrement important sous les vêtements et un manque d'élasticité du dispositif de support de la minerve; en outre, ces corsets enserrent rigidement le tronc, ce qui nuit à la mobilité de ce dernier, notamment dans la direction latérale et d'avant en arrière.

C'est ainsi que le brevet français 1.037.220 décrit un appareil orthopédique comprenant une ceinture en métal flexible entourant la taille, dont les extrémités antérieures sont écartées et sur lesquelles sont fixées, à l'avant une plaque verticale rigide, destinée à comprimer le bas-ventre et, à l'arrière, deux lames verticales flexibles en métal, transmettant les pressions respectivement à une plaque dorsale et une plaque sacrée. Un tel appareil soulage le dos en soutenant le poids du ventre, mais ne permet aucun redressement de la colonne vertébrale par suite de la flexibilité des éléments reliant les plaques entre elles.

L'invention faisant l'objet de la présente invention permet d'éviter ces inconvénients.

Elle consiste en un appareil orthopédique pour le redressement de la colonne vertébrale comprenant:

— une ceinture pelvienne destinée à entourer le corps du patient, ladite ceinture présentant une partie surélevée postérieure formant dossier et deux extrémités antérieures espacées l'une de l'autre de manière à ménager une ouverture centrale;

— une plaque adbominale rigide aux angles arrondis dont l'extrémité inférieure se fixe sous les extrémités antérieures de la ceinture pelvienne et qui présente dans sa partie supérieure une concavité tournée vers l'estomac du patient, ledit appareil étant caractérisé en ce que:

— la ceinture pelvienne est rigide et conformée de manière à entourer le bassin du patient et prendre appui sur l'un au moins des trochanters de celui-ci;

— la plaque abdominale a une forme trapézoïdale et est disposée de telle sorte que la petite base du trapèze soit située vers le bas et la grande base arrive à hauteur de l'appendice xyphoïde du patient;

— il comporte en outre:

deux coquilles latérales rigides ayant chacune une forme générale en U échancrée au milieu, en haut et en bas pour épouser l'un des côtés du patient en chevauchant la crête iliaque, leur partie inférieure présentant un rebord ou un découpage plus ou moins prononcé pour prendre appui ou chevaucher la crête iliaque, les dites coquilles ayant des parties antérieures respectives qui sont de hauteurs sensiblement similaires et inférieures à celle de la plaque abdominale et sont fixées toutes deux sur la partie médiane ou supérieure de la plaque abdominale, et des parties postérieures qui sont de hauteurs sensiblement supérieures à celles de leurs parties antérieures respectives, et sont fixées toutes deux sur la partie surélevée postérieure formant dossier de la ceinture pelvienne lesdites ceintures plaque et coquilles étant fixées entre elles de manière réglable pour permettre un réglage progressif de la correction souhaitée.

Une telle structure facilite la mise en place de l'appareil par un montage des différents éléments constitutifs directement sur le malade; elle assure une meilleure adaptation de ces éléments à la morphologie de chaque individu; elle permet un réglage progressif de la ou des corrections souhaitées par déplacement relatif de divers éléments entre eux; et enfin, on peut y adapter soit des minerves de type connu soit, lorsque le malade est allongé, un système de traction par sangles, pour provoquer une élongation de la colonne vertébrale.

La présente invention va être décrite en détail à l'aide des dessins suivants, dans lesquels:

— la figure 1 représente une première réalisation préférée de l'appareil selon l'invention vue de face;
— la figure 2 est une vue éclatée des différents éléments de la réalisation de la figure 1;
— la figure 3 est une vue de profil de la plaque abdominale représentée sur les figures 1 et 2 et montrant son profil en S;
— la figure 4 est une vue arrière de la réalisation des figures 1 et 2.
— les figures 5 et 6 représentent une variante selon l'invention vue respectivement de face et de dos;
— la figure 7 est une vue éclatée des éléments de l'appareil représenté sur les figures 5 et 6;
— la figure 8 représente une autre variante de l'invention et la figure 9 des éléments de cette dernière;
— la figure 10 représente l'appareil selon l'invention avec adjonction d'une minerve;
— les figures 11 et 12 représentent respectivement une vue partielle découpée et une coupe selon la ligne A—A' de la figure 11, du dispositif de support de la minerve, à une échelle agrandie;
— la figure 13 représente une variante d'un tel dispositif de support;
— la figure 14 illustre différents détails de construction de ces dispositifs de support;
— les figures 15 à 21 représentent différents modes de fixation du support de la minerve à l'appareil selon l'invention; et
— les figures 22 et 23 représentent des exemples de moyens de fixation des différents éléments de l'appareil selon l'invention entre eux.

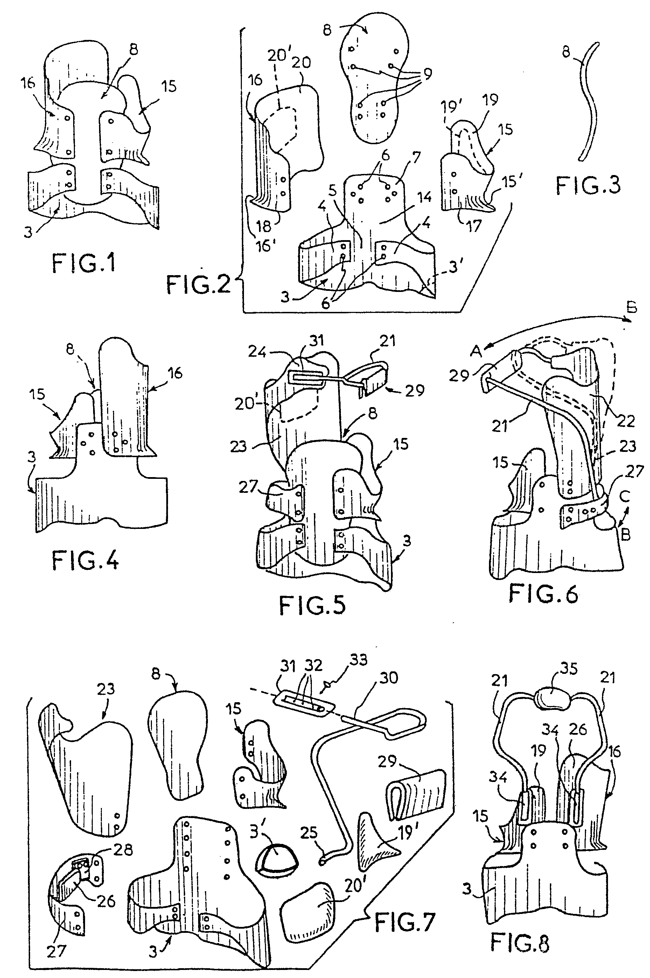

La ceinture pelvienne 3 est l'élément de base de l'appareil selon l'invention; elle consiste comme le montrent les figures 1 et 2 en un élément rigide 3 de forme généralement circulaire, qui entoure le bassin en moulant les masses fessières et qui prend appui sur l'un au moins des trochanters pour se terminer par deux extrémités antérieures 4 espacées l'une de l'autre de manière à ménager une ouverture 5.

Cette ouverture peut permettre au patient d'enfiler la ceinture lorsque celle-ci est d'un seul tenant. La ceinture peut toutefois se composer de deux éléments latéraux rigides reliés entre eux le long d'une ligne verticale située à l'opposé de l'ouverture 5 pour permettre à la personne recevant l'appareil d'enfiler la ceinture soit par devant par l'ouverture 5, soit par derrière où les deux éléments latéraux sont alors reliés entre eux avec recouvrement le long de la ligne verticale.

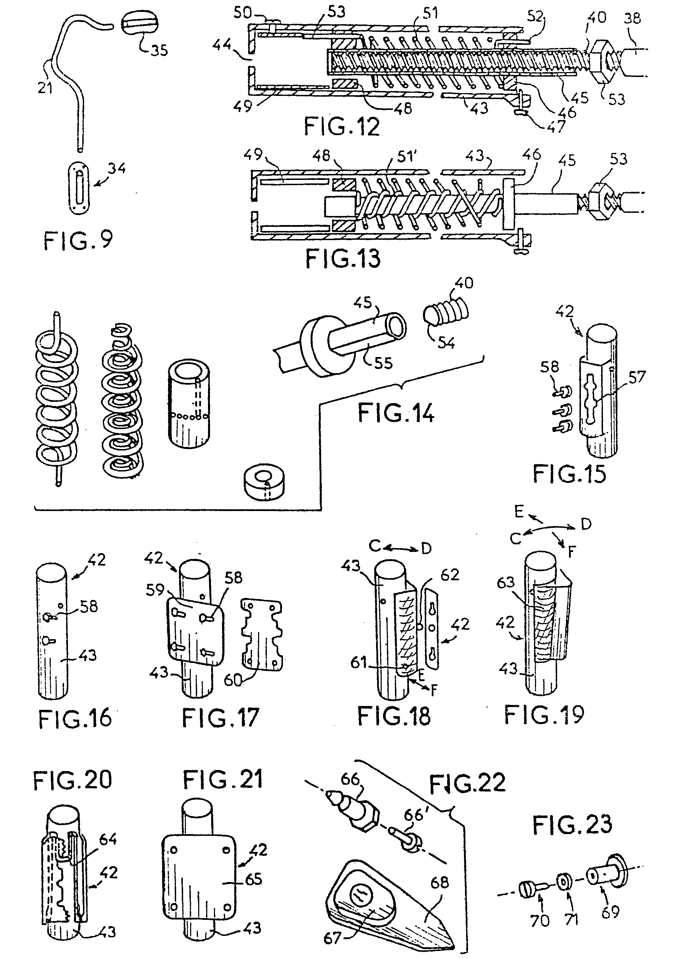

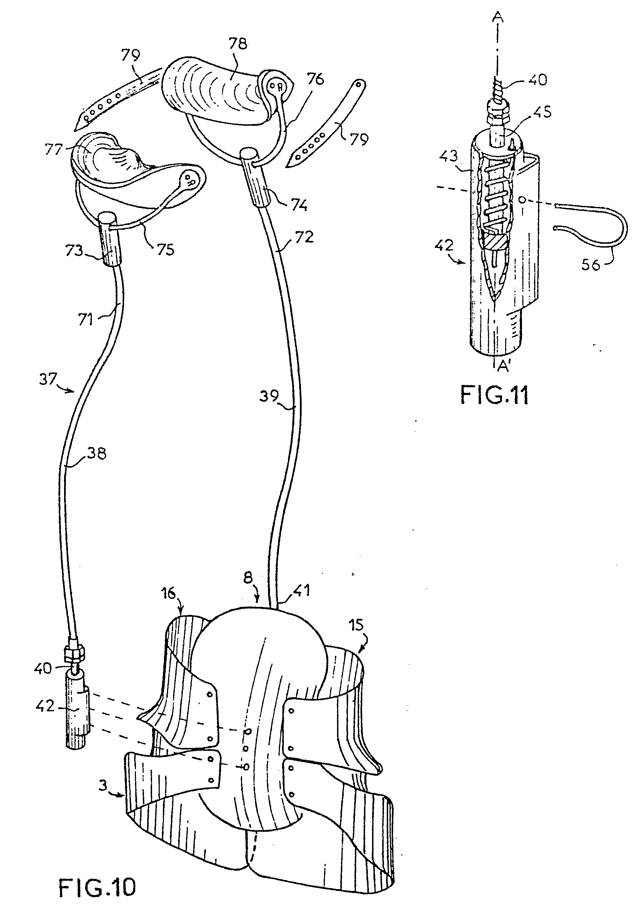

La partie centrale postérieure 14 de la ceinture et ses deux extrémités antérieures 4 sont munies d'un ou plusieurs jeux de perforation 6 permettant d'y faire passer des moyens de fixation connus, notamment du genre tenons à gorge avec boutons-pressions ou tenons à vis représentés respectivement sur les figures 22 et 23 pour y fixer les autres éléments de l'appareil selon l'invention.

La ceinture, qui a la forme d'un anneau non fermé sur le devant, présente de préférence une partie centrale postérieure surélevée 7 formant dossier, qui sert à la fixation des autres éléments de l'appareil à savoir les coquilles latérales lombaire et dorsale.

La plaque abdominale 8 sert de pièce de liaison aux différents éléments constituant l'appareil selon l'invention; elle consiste en un élément rigide de forme générale trapézoïdale aux angles arrondis qui, comme le montrent notamment les figures 1 et 10, est recouverte par les extrémités antérieures 4 de la ceinture pelvienne 3 à sa partie inférieure; sa partie supérieure présente notamment une concavité tournée vers l'estomac du patient et elle se termine à hauteur de l'appendice xyphoïde pour dégager la cage thoracique du patient.

Cette plaque présente un ou plusieurs jeux de perforations 9 dans sa partie inférieure pour permettre sa fixation à la ceinture pelvienne 3 et dans sa partie supérieure pour permettre celle des coquilles latérales à l'aide de moyens de fixation classiques.

L'appareil selon l'invention comporte également deux coquilles latérales 15 et 16 qui recouvrent les flancs du patient et se présentent chacune sous forme d'un élément rigide ayant la forme générale d'un U, échancré au milieu en haut et en bas pour épouser l'un des côtés du patient en chevauchant sur la crête iliaque, la partie inférieure présentant soit un rebord 15' ou 16', soit un découpage plus ou moins prononcé pour prendre appui ou chevaucher la crête iliaque. C'est ainsi que les deux coquilles latérales peuvent présenter un tel rebord ou un tel découpage ou bien l'une présenter ledit rebord et l'autre ledit découpage.

Ces deux coquilles présentent chacune une partie antérieure 17, 18 et une partie postérieure 19, 20, qui sont fixées respectivement sur la partie supérieure de la plaque abdominale 8 et sur la partie postérieure centrale de la ceinture pelvienne 3 et notamment sur la partie surélevée formant dossier de cette dernière.

La hauteur de la partie antérieure 17, 18 de chacune des deux coquilles 15, 16 est sensiblement la même et inférieure au sommet de la plaque abdominale 8, lorsque ces différents éléments sont mis en place sur le patient, mais la hauteur de leurs parties postérieures 19, 20 est sensiblement supérieure à celle de leurs parties antérieures respectives.

C'est ainsi que l'une des coquilles 15 peut soutenir la courbure du dos du patient correspondant aux vertèbres lombaires alors que l'autre coquille 16 a sa partie postérieure qui s'étend jusqu'aux vertèbres dorsales, comme illustré sur les figures 1 et 5 pour corriger la courbure dorsale de la colonne vertébrale ou servir de contre-appui à la coquille lombaire 15.

Dans une autre forme de réalisation représentée sur la figure 10, les deux coquilles 15, 16 peuvent être de mêmes dimensions, pour soigner une hyper-lordose ou une cyphose.

Selon une autre variante, non représentée sur les figures, un élément supplémentaire peut relier une coquille latérale lombaire à l'autre coquille latérale dorsale. Un tel élément a par exemple la forme d'un L renversé dont la branche verticale part de la coquille latérale lombaire à sa partie inférieure et la branche horizontale rejoint la coquille latérale dorsale à sa partie supérieure; cet élément sert alors à soutenir le côté du patient opposé à celui sur lequel s'appuie la coquille latérale dorsale.

Selon d'autres réalisations tombant dans le cadre de la présente invention, on peut encore ajouter d'autres éléments complémentaires selon la déformation de la colonne vertébrale du patient, notamment en cas de courbure haute de cette dernière.

Les différents éléments de l'appareil peuvent être réalisés ou préfabriqués en une matière quelconque assurant la rigidité nécessaire à l'obtention des corrections envisagées, tels que matière plastique, matière métallique ou matière naturelle. Ils possèdant une épaisseur plus ou moins forte selon le degré de rigidité et les poussées plus ou moins fortes à exercer en un ou plusieurs points précis du corps du patient.

Les matières premières préférées pour la fabrication des différents éléments de l'appareil selon l'invention sont les matières plastiques et en particulier celles choisies dans le groupe comprenant le polyéthylène, le polypropylène, le nylon et le copolymère acrylonitrilepolyméth-acrylate de méthyle.

On les utilise sous forme de plaques, ayant une épaisseur comprise généralement entre 2 et 5 mm et de préférence entre 3 et 4 mm, qui sont découpées et formées en fonction de la morphologie du patient et permettent d'obtenir des éléments flexibles.

On peut également prévoir des pièces d'appui ou de renforcement se présentant notamment sous forme d'éléments gonflables ou d'éléments en mousse à alvéoles fermées, de forme, qualité et épaisseur convenables, qui sont disposées en des emplacements judicieusement choisis pour renforcer l'action corrective des différents éléments de l'appareil.

C'est ainsi qu'on peut prévoir une telle pièce 3' sur l'appui trochanter de la ceinture pelvienne, représentée en traits pointillés sur la figure 2; une autre pièce d'appui 19' de forme générale triangulaire peut être disposée sur la coquille lombaire 15 avec un côté vertical qui repousse la courbure de la colonne vertébrale à corriger; enfin, une autre pièce d'appui 20', par exemple de forme quadrangulaire, peut se trouver à l'intérieur de la coquille 16 (figure 2) ou d'un élément de cette dernière 23 (figure 5) pour renforcer l'appui sur une gibbosité dorsale.

Dans l'appareil selon l'invention, tous les éléments, à savoir la ceinture pelvienne, la plaque abdominale et les deux coquilles latérales, une fois mises en place, coopèrent pour provoquer l'érection du rachis avec les poussées latérales nécessaires pour effectuer les corrections de la colonne vertébrale, selon les cas cliniques.

L'appareil peut également comporter un certain nombre d'éléments complémentaires, tels que des tiges métalliques pour agir sur des gibbosités et redresser la colonne vertébrale, un dispositif d'accrochage de sangles situé sur la ceinture pelvienne et monté sur des lits ou des tables d'élongation, un dispositif occipito-mentonnier pour produire une élongation continue ou intermittente de la colonne vertébrale, ou tout autre élément ou combinaison d'éléments, connu et utilisé en orthopédie.

On peut en effet prévoir l'adjonction d'une ou plusieurs ferrures, tiges métalliques ou barres d'acier 21 ou en toute matière présentant les mêmes résistance, ténacité et élasticité, dont les extrémités reposent dans des gouttières ménagées sur des parties de deux éléments différents de l'appareil se trouvant d'un même côté du corps du patient, cette tige prenant appui entre ses deux extrémités sur le côté opposé du patient.

Par exemple, comme le montrent les figures 5 et 6, une tige métallique 21 peut prendre appui horizontalement sur le côté droit de la ceinture pelvienne, s'élever pratiquement verticalement jusqu'à hauteur d'une gibbosité à redresser en appuyant sur la partie postérieure 22 d'une coquille dorsale disposée sur le côté droit, partir en diagonale dans le dos, passer sous l'aisselle gauche et se terminer pratiquement à l'horizontale sur une prolongation antérieure élevée 24 de cette coquille dorsale.

Dans une telle réalisation, la coquille dorsale se compose de deux éléments 23 et 27, l'élément inférieur 27 comportant, comme le montre la figure 7, une gouttière 26, située vers l'intérieur, qui reçoit l'extrémité inférieure de la tige métallique 21. Cette extrémité de la tige 21 se trouve calée dans la gouttière 26 par une cale 28, pour régler la rotation de la partie supérieure du tronc dans la direction CB (figure 6).

Une protection flottante 29, placée sur la tige à l'endroit où elle est en contact avec le corps sous l'aisselle est représentée sur la figure 7. L'autre extrémité 30 de la tige est perforée et s'enfile dans une gouttière 31 en forme de glissière tubulaire munie de trous 32 et disposée sur la prolongation antérieure 24 de l'élément 23 de la coquille dorsale droite. Une cheville 33, mettant en correspondance les perforations de la tige et de la glissière, permet un réglage en fonction de la morphologie du patient. Il est à remarquer qu'un tel dispositif permet au malade de s'incliner latéralement aussi bien à gauche qu'à droite (flèche AB), en agissant par appui sur la gibbosité avec dérota-tion.

Dans une autre forme de réalisation représentée sur la figure 8, une première tige ou barre métallique 21 part d'un logement vertical 34 également en forme de glissière tubulaire situé dans la partie postérieure 19 d'une des coquilles latérales 15 où son extrémité est simplement enfilée, passe sous l'aisselle correspondante puis devant la cage thoracique où elle se termine sur un appui sternal 35; de même une autre tige métallique 21 part du même appui sternal 35, passe sous l'autre aisselle et se termine dans un logement vertical analogue 34 situé dans la partie postérieure 26 de l'autre coquille 16.

Le dispositif occipito-mentonnier ou minerve que l'on peut adapter sur l'appareil selon l'invention peut être d'un type déjà connu comme ceux décrits dans les brevets français n° 1 276 078, n° 2 031 200 ou n° 2 349 321, ou comme ceux utilisés dans les dispositifs Mil-waukee ou d'un type nouveau qui va être maintenant décrit en détail et qui a l'avantage sur les précédents de combiner une grande simplicité de structure avec une plus grande liberté de mouvement pour le patient.

La minerve désignée par la référence générale 37 est constituée essentiellement de deux tiges montants verticales 38, 39 disposées dans le plan de symétrie du patient, filetées au moins à leurs extrémités inférieures 40, 41 où elles s'insèrent dans un dispositif de support 42 accroché à l'un des éléments de l'appareil selon l'invention, et dont les extrémités supérieures 71, 72 sont reliées respectivement au moyen d'une bague 73, 74 et d'un arceau 75, 76 à un appui mentonnier 77 et à une têtière 78 reliés entre eux par des pattes 79.

L'appui mentonnier 77 présente de préférence une surface rembourrée, par exemple par de la mousse, et façonnée de manière à épouser la machoire du patient.

Une réalisation particulière du dispositif de support 42 de l'extrémité inférieure filetée de chaque tige montant est illustrée en détail sur les figures 11 et 12. Ce dispositif 42 comporte un cylindre 43 solidaire d'une base en U possédant des moyens de fixation classiques sur un élément de l'appareil selon l'invention, par exemple sur la ceinture pelvienne 3 et la plaque abdominale 8.

Ce cylindre 43, généralement fermé à sa partie inférieure ou présentant un trou taraudé 44, reçoit un tube 45 muni d'une collerette 46. Ce tube 45, maintenu à l'intérieur du cylindre 43 par un moyen de blocage tel une vis 47, coulisse dans une bague de guidage 48 reposant librement sur une bague de course 49 fixée d'une manière réglable à la paroi du cylindre 43 par une vis 50. Un ressort de compression et de torsion 51, dont une extrémité 52 est fixée à la collerette 46 et l'autre extrémité 53 est fixée à la bague de course 49 après avoir traversé la bague de guidage 48, sollicite en permanence le tube 45 vers son extrémité supérieure ouverte en faisant buter la collerette 46 sur la vis de blocage 47.

L'extrémité filetée 40 (ou 41) de la tige-montant 38 (ou 39) présente un moyen empêchant sa rotation dans le tube 45, tel un méplat 54 et elle est enfilée dans le tube 45, qui présente un méplat correspondant 55.

La tige 38, comme la tige 39, reposent sur l'extrémité du tube par un ou plusieurs écrous 53 dont on peut régler la position sur la partie filetée 40 (ou 41) de la tige.

On peut également ajouter au ressort 51 un autre ressort 51' enfilé autour du premier et de pas contraire pour effectuer une poussée plus active (voir figure 13).

Un clip de blocage 56 amovible peut s'enfiler transversalement dans le cylindre 43 pour pouvoir effectuer éventuellement un traitement intermittent.

Un tel dispositif de support télescopique de la minerve permet de régler la traction et la rotation exercées sur la colonne vertébrale, en laissant au patient le maximum de liberté et en maintenant un effet progressif.

Il est en effet important d'éviter une action de traction longitudinale trop prolongée ou de torsion trop poussée qui produit une réaction du malade provoquant souvent un problème dentaire.

C'est pourquoi on prévoit un réglage des courses des ressorts tant dans le sens de la poussée verticale qui dans le sens de la rotation.

Ces dispositifs de support 42 de la minerve 37 s'accrochent sur la plaque abdominale 8 et sur la partie postérieure surélevée formant dossier 7 de la ceinture pelvienne par tous moyens illustrés notamment sur les figures 15 à 21.

La figure 15 représente un système de fixation amovible des supports 42 par une glissière à encoches 57 ménagée dans une bague en U et pouvant recevoir dans tenons 58; la figure 16 représente le cas inverse où les tenons sont solidaires du cylindre 43; la figure 17 montre une plaque 59 solidaire du cylindre 43 et munie de tenons 58 qui peuvent être accrochés sur les éléments de l'appareil muni d'éléments d'accrochage correspondants, tels qu'une glissière 60.

Selon la réalisation représentée à la figure 18, le moyen d'accrochage et de décrochage amovible du dispositif de support de la minerve comporte un système à pivot permettant des rotations selon un premier axe horizontal 61 et un deuxième axe horizontal 62 perpendiculaire au premier et situé dans le plan de symétrie du patient; un tel système permet des inclinaisons dans deux directions perpendiculaires CD et EF.

Le même résultat peut être obtenu en noyant le cylindre 43 dans une masse élastique 63 comme représenté sur la figure 19.

La figure 20 montre que ces différents moyens de fixation peuvent comprendre un ressort amovible 64 permettant le blocage des dispositifs 42 dans leur position d'accrochage.

La figure 21 montre un cylindre 43 solidaire d'une plaque 65 muni de simples perforations pour le passage du moyen de fixation du même type que ceux utilisés pour fixer entre eux les différents éléments de l'appareil selon l'invention.

De tels moyens de fixation comprennent, par exemple, des tenons à gorge 66 fixés à l'aide d'une vis 66' et sur lesquels on peut enfiler un bouton-pression 67 fixé sur une languette 68 (figure 22) et/ou un système de tenons 69 à vis 70 et rondelle 71 (figure 23).

**Revendications**

1. Appareil orthopédique pour le redressement de la colonne vertébrale comprenant:

— une ceinture pelvienne (3) destinée à entourer le corps du patient, ladite ceinture présentant une partie surélevée postérieure formant dossier (7) et deux extrémités antérieures (4) espacées l'une de l'autre de manière à ménager une ouverture centrale (5);

— une plaque abdominale rigide (8) aux angles

**0 016 293**

arrondis dont l'extrémité inférieure se fixe sous les extrémités antérieures (4) de la ceinture pelvienne et qui présente dans sa partie supérieure une concavité tournée vers l'estomac du patient, ledit appareil étant caractérisé en ce que;

— la ceinture pelvienne est rigide et conformée de manière à entourer le bassin du patient et prendre appui sur l'un au moins des trochanters de celui-ci;

— la plaque abdominale (8) a une forme trapézoïdale et est disposée de telle sorte que la petite base du trapèze soit située vers le bas et la grande base arrive à hauteur de l'appendice xyphoïde du patient;

— il comporte en outre:

deux coquilles latérales rigides (15, 16) ayant chacune une forme générale en U échancrée au milieu, en haut et en bas pour épouser l'un des côtés du patient en chevauchant la crête iliaque, leur partie inférieure présentant un rebord (15' et 16') ou un découpage plus ou moins prononcé pour prendre appui ou chevaucher la crête iliaque, les dites coquilles ayant des parties antérieures respectives (17 et 18) qui sont de hauteurs sensiblement similaires et inférieures à celle de la plaque abdominale et sont fixées toutes deux sur la partie médiane ou supérieure de la plaque abdominale, et des parties postérieures (19 et 20) qui sont de hauteurs sensiblement supérieures à celles de leurs parties antérieures respectives, et sont fixées toutes deux sur la partie surélevée postérieure formant dossier (7) de la ceinture pelvienne (3) lesdites ceinture (3), plaque (8) et coquilles (15 et 16) étant fixées entre elles de manière réglable pour permettre un réglage progressif de la correction souhaitée.

2. Appareil selon la revendication 1, caractérisé en ce que la ceinture pelvienne (3) consiste en deux éléments latéraux rigides reliés entre eux le long d'une ligne verticale située à proximité de l'axe de la partie surélevée formant dossier (7).

3. Appareil selon les revendications 1 ou 2, caractérisé en ce que les parties postérieures des deux coquilles latérales sont de hauteurs identiques.

4. Appareil selon les revendications 1 ou 2, caractérisé en ce que les parties postérieures (19 et 20) des deux coquilles latérales (15 et 16) sont de hauteurs différentes, l'une s'élevant uniquement le long des vertèbres lombaires et l'autre s'élevant jusqu'à hauteur des vertèbres dorsales, ces deux coquilles latérales étant éventuellement reliées entre elles par un élément supplémentaire en forme de L renversé et accessoirement avec combinaison d'éléments connus en orthopédie, entre autres appuis claviculaires ou sternal.

5. Appareil selon l'une quelconque des revendications 1 à 4, caractérisé en ce que l'une des coquilles latérales rigides se compose de deux éléments (23 et 27), dont l'élément inférieur (27) comporte une gouttière (26) située vers l'intérieur de l'appareil.

6. Appareil selon l'une quelconque des revendications 1 à 5, caractérisé en ce qu'il comprend également une tige métallique rigide (21) dont une extrémité repose sur une gouttière fixée sur une partie d'un premier élément de l'appareil et l'autre extrémité sur une gouttière fixée sur une partie d'un deuxième élément de l'appareil, cette tige prenant appui entre ses deux extrémités sur la partie du corps du patient opposée à celle sur laquelle se trouvent lesdites parties des premier et second éléments.

7. Appareil selon la revendication 6, caractérisé en ce que la tige métallique rigide (21) prend appui sur une gibbosité située du côté du patient où se trouvent les extrémités de ladite tige.

8. Appareil selon la revendication 6, caractérisé en ce que la gouttière recevant une extrémité de la tige métallique rigide se présente sous forme d'une glissière tubulaire.

9. Appareil selon l'une quelconque des revendications précédentes, caractérisé en ce qu'un ou plusieurs des éléments de l'appareil comprennent des pièces d'appui ou de renforcement (3', 19' ou 20') en mousse à alvéoles fermées destinées à renforcer l'action corrective desdits éléments sur la colonne vertébrale.

10. Appareil selon l'une quelconque des revendications 1 à 9, caractérisé en ce que les moyens de fixation des divers éléments de l'appareil entre eux consistent en des tenons à gorge (66) pour bouton-pression et/ou des tenons à vis (69, 70), dont le diamètre extérieur correspond au diamètre intérieur des perforations prévues dans les différents éléments de l'appareil.

11. Appareil selon l'une quelconque des revendications 1 à 10, caractérisé en ce qu'il comprend également un support occipito-mentonnier (37) relié à des points d'appui disposés sur un ou plusieurs éléments de l'appareil.

12. Appareil selon la revendication 11, caractérisé en ce que le support occipito-mentonnier (37) comporte deux tiges-montants filetées verticales (38, 39) situées dans le plan de symétrie du patient et dont l'extrémité supérieure est reliée respectivement au moyen d'une bague et d'un arceau à un appui mentonnier (77) et à une têtière (78) montés à pivot et dont les extrémités inférieures reposent chacune grâce à un ou plusieurs écrous de réglage de la hauteur (53), dans un dispositif télescopique (42) fixé à la ceinture pelvienne.

13. Appareil selon la revendication 12, caractérisé en ce que chaque dispositif télescopique (42) comprend un tube creux (45) recevant l'extrémité d'une tige-montant filetée et pourvu d'une collerette (46) et d'une bague de course (49) entre lesquels est inséré au moins un ressort (51) et une bague de guidage (48), le tube et les deux bagues étant disposées à l'intérieur d'un cylindre (43) solidaire d'un système de fixation monté sur la ceinture pelvienne.

14. Appareil selon la revendication 13, caractérisé en ce que chaque cylindre (43) des dispositifs télescopiques est fixé à la ceinture par un système d'accrochage comportant un pivot permettant des rotations du cylindre autour de deux axes horizontaux perpendiculaires dont l'un est situé, de préférence de manière amovible, dans le plan de symétrie du patient.

15. Appareil selon la revendication 13 ou 14, caractérisé en ce que les extrémités du ressort (51) du dispositif télescopique sont rendues solidaires respectivement de la collerette (46) et du cylindre (43) et que l'extrémité de la tige-montant filetée et celle du tube recevant cette dernière comportent toutes deux un moyen (54, 55) empêchant la rotation de la tige par rapport au tube.

**Patentansprüche**

1. Orthopädischer Apparat zur Behandlung der Wirbelsäule mit:

— einem Beckengürtel (3), bestimmt, den Körper des Patienten zu umschlingen, wobei der Gürtel einen hochgezogenen hinteren Abschnitt besitzt, der das Rückenteil (7) bildet, und zwei voneinander einen Abstand aufweisende vordere Ende (4) in der Weise, daß eine zentrale Öffnung (5) gebildet ist;

— eine steife Bauchplatte (8) mit abgerundeten Ecken, deren unteres Ende unter den vorderen Enden (4) des Beckengürtels festliegt und die in ihrem oberen Teil eine dem Magen des Patienten zugewendete Ausnehmung zeigt, dadurch gekennzeichnet, daß:

— der Beckengürtel steif ist und so ausgebildet, daß er das Becken des Patienten umschlingt und sich an mindestens einem Trochanter (Rollhügel des Hüftbeins) desselben abstützt;

— die Bauchplatte (8) trapezförmig ist und so angeordnet, daß die kleine Seitenlinie des Trapezes an der Unterseite gelegen ist und die große Seitenlinie bis zur Höhe des appendix xyphoidus (Brustbeinende) des Patienten reicht;

— er weiter enthält:

zwei steife Seitenschalen (15, 16), die jeweils eine etwa U-förmige, in der Mitte, oben und unten ausgeschnittene Form besitzt zur Anpassung an eine der Körperseiten des Patienten, und den Hüftbeingrat überdecken, wobei ihr unterer Teil jeweils einen Umschlag (15' und 16') oder einen mehr oder weniger stark ausgebildeten Einschnitt zeigt, um auf den Hüftbeingrat aufzustützen oder aufzusitzen, wobei die jeweiligen vorderen Seiten (17 und 18) der Seitenschalen im wesentlichen gleichartige Höhen, kleiner als die der Bauchplatte besitzen und beide auf dem mittleren oder oberen Abschnitt der Bauchplatte befestigt sind, und hintere Abschnitte (19 und 20) von wesentlich größerer Höhe als die ihrer jeweiligen vorderen Teile und beide an dem hochgezogenen hinteren, das Rückenteil (7) bildenden Abschnitt des Beckengürtels (3) befestigt sind, und wobei der Gürtel (3), die Platte (8) und die Schalen (15 und 16) untereinander auf einstellbare Weise befestigt sind, um eine fortschreitende Regelung der erwünschten Korrektur zu ermöglichen.

2. Apparat nach Anspruch 1, dadurch gekennzeichnet, daß der Beckengürtel (3) aus zwei seitlichen starren Elementen besteht, die miteinander längs einer vertikalen Linie verbunden sind, die in der Nähe der Achse des hochgezogenen, das Rückenteil (7) bildenden Abschnittes liegt.

3. Apparat nach Anspruch 1 oder 2, dadurch gekennzeichnet, daß die hinteren Abschnitte der beiden Seitenschalen von identischer Höhe sind.

4. Apparat nach Anspruch 1 oder 2, dadurch gekennzeichnet, daß die hinteren Abschnitte (19 und 20) der beiden Seitenschalen (15 und 16) von unterschiedlicher Höhe sind, daß die eine sich nur bis zur Höhe der Lendenwirkbeln erhebt und die andere sich bis zur Höhe der Rückenwirbel erhebt, wobei die beiden Seitenschalen ggf. miteinander durch ein Zusatzelement in Form eines umgekehrten L und zusätzlich mit einer Kombination von in der Orthopädie bekannten Elementen, u.a. Schlüsselbein- oder Bruststützen verbunden sind.

5. Apparat nach einem der Ansprüche 1 bis 4, dadurch gekennzeichnet, daß eine der starren Seitenschalen sich aus zwei Elementen (23 und 27) zusammensetzt von denen das untere Element (27) eine an der Innenseite des Apparates gelegene Schiene (26) trägt.

6. Apparat nach einem der Ansprüche 1 bis 5, dadurch gekennzeichnet, daß er gleichermaßen eine starre Metallstange (21) aufweist, deren eines Ende an einem Abschnitt eines ersten Elementes des Apparates befestigt und dessen anderes Ende an einer Schiene befestigt ist, die an einem Abschnitt eines zweiten Elementes des Apparates befestigt ist, wobei die Stange zwischen ihren Enden an einem Abschnitt des Körpers des Patienten aufliegt, der dem Körperabschnitt gegenüberliegt, an dem sich die genannten Abschnitte des ersten und des zweiten Elementes befinden.

7. Apparat nach Anspruch 6, dadurch gekennzeichnet, daß die starre Metallstange (21) sich an einem an der Seite des Patienten gelegenen Buckel abstützt, an der sich die Enden der Stange befinden.

8. Apparat nach Anspruch 6, dadurch gekennzeichnet, daß die ein Ende der starren Metallstange aufnehmende Schiene sich in Form eines rohrförmigen Gleitstükkes zeigt.

9. Apparat nach einem der vorangehenden Ansprüche, dadurch gekennzeichnet, daß ein oder mehrere Element(e) des Apparates Auflage- oder Verstärkungsstücke (3', 19' oder 20') aus einem geschlossenzelligen Schaumstoff aufweisen, die dazu bestimmt sind, die Korrekturwirkung der Elemente auf die Rückwirbelsäule zu verstärken.

10. Apparat nach einem der Ansprüche 1 bis 9, dadurch gekennzeichnet, daß die Befestigungsmittel für die verschiedenen Elemente des Apparates untereinander Kehlstifte (66) für Druckknopfverbindung und/oder Schraubbolzen (69, 70) enthalten, deren Außendurchmesser dem Innendurchmesser der Durchbrüche entspricht, die in den verschiedenen Elementen des Apparates vorgesehen sind.

11. Apparat nach einem der Ansprüche 1 bis 10, dadurch gekennzeichnet, daß gleichermaßen eine Hinterkopf-Kinn-Stütze (37) vorgesehen ist, die mit Auflagestellen verbunden ist, die sich an einem oder mehreren Elementen des Apparates befinden.

12. Apparat nach Anspruch 11, dadurch gekennzeichnet, daß die Hinterkopf-Kinn-Stütze (37) zwei vertikale mit Gewinde versehene Stützstangen (38, 39) aufweist, die in der Symmetrieebene des Patienten liegen und deren oberes Ende jeweils mittels einer Öse und eines Bügels mit einer Kinnstütze (77) und einer Kopfstütze (78) verbunden ist, die schwenkbar befestigt sind und deren untere Endstücke mittels einer oder mehrerer Stellmuttern (53) für die Höhe in einer Teleskopvorrichtung (42) ruhen, welche am Beckengürtel befestigt ist.

13. Apparat nach Anspruch 12, dadurch gekennzeichnet, daß jede Teleskopvorrichtung (42) ein Hohlrohr (45) aufweist, welches das Ende einer mit Gewinde versehenen Stützstange aufnimmt und mit einem Bund (46) und einem Schubring (49) versehen ist, zwischen denen wenigstens eine Feder (51) und ein Führungsring (48) eingesetzt sind, wobei das Rohr und die beiden Ringe im Inneren eines Zylinders (43) angeordnet sind, der einstückig mit einem am Beckengürtel angebrachten Befestigungssystem ausgeführt ist.

14. Apparat nach Anspruch 13, dadurch gekennzeichnet, daß jeder Zylinder der Teleskopvorrichtungen am dem Gürtel über ein Verankerungssystem angebracht ist, das Schwenkbewegungen des Zylinders um zwei aufeinander senkrecht stehende horizontale Achsen erlaubt, von denen eine, vorzugsweise unbewegbar, in der Symmetrieebene des Patienten liegt.

15. Apparat nach Anspruch 13 oder 14, dadurch gekennzeichnet, daß die Enden der Feder (51) der Teleskopvorrichtung jeweils mit dem Bund (46) bzw. dem Zylinder (43) fest verbunden sind, und daß das Ende der mit Gewinde versehenen Stützstange und das dieses Ende aufnehmende Ende des Rohres beide ein Mittel (54, 55) besitzen, welches die Schwenkung der Stange bezüglich des Rohres verhindert.

## Claims

1. An orthopedic apparatus for straightening a vertebral column comprising:

— a pelvian belt (3) for surrounding the body of the patient, said belt comprising a posterior raised portion forming a back (7) and two anterior ends (4) spaced from one another so as to provide a central opening (5) therebetween;
— a rigid abdominal plate (8) with rounded angles and the lower end of which is secured under the anterior ends (4) of the pelvian belt and which comprises in its upper portion a concavity turned to the patient's stomach, said apparatus being characterized in that:
— the pelvian belt is rigid and configured so as to surround the patient's pelvis and take rest on at least one of the patient's trochanters;
— the abdominal plate (8) is of a trapezoidal shape and disposed such that the smaller basis of the trapezium is located downwardly and the larger basis reaches the level of the patient's xyphoid appendix;
— it comprises moreover:

two rigid lateral shells (15, 16) each having a general U-shape notched in the center, upwardly and downwardly to follow one side of the patient by riding on the iliac crest, their lower portion presenting a flange (15' and 16') or a more or less pronounced cutting in to find support or ride on the iliac crest, said shells having respective anterior portions (17 and 18) which are of a substantially similar heights lower than that of the abdominal plate and are both fixed to the median or upper portion of the abdominal plate, and posterior portions (19 and 20), the heights of which are substantially higher than those of their respective anterior portions and both of which are fixed to the posterior raised portion forming a back (7) of the pelvian belt (3), said belt (3), plate (8) and shells (15 and 16) being secured to one another in an adjustable manner to permit progressive adjustment of the desired correction.

2. An apparatus according to claim 1, characterized in that the pelvian belt (3) consists of two rigid lateral elements connected to one another along a vertical line located close to the axis of the back-forming raised portion (7).

3. An apparatus according to claims 1 or 2, characterized in that the posterior portions of both lateral shells are of identical heights.

4. An apparatus according to claims 1 or 2, characterized in that the posterior portions (19 and 20) of both lateral shells (15 and 16) are of different heights, with the one only rising along the lumbar vertebrae and the other rising up to the height of the dorsal vertebrae, these two lateral shells possibly being connected to one another through a reversed L-shaped supplementary element and subsidiarily with a combination of elements known in orthopedia, among other clavicular or sternal rests.

5. An apparatus according to any one of claims 1 to 4, characterized in that one of the rigid lateral shells is composed of two elements (23 and 27), the lower element (27) of which comprises a gutter (26) located inwardly of the apparatus.

6. An apparatus according to any one of claims 1 to 5, characterized in that it also comprises a rigid metallic rod (21), one end of which lies on a gutter secured to a portion of a first element in the apparatus, and the other end of which rests on a gutter secured to a portion of a second element in the apparatus, such rod taking rest between both of its ends on that portion of the patient's body opposed from that on which said portions of the first and second elements lie.

7. An apparatus according to claim 6, characterized in that the rigid metallic rod (21) rests on a gibbosity lying on that side of the patient where the ends of said rod lie.

8. An apparatus according to claim 6, characterized in that the gutter receiving one end of the rigid metallic rod is in the form of a tubular slideway.

9. An apparatus according to any one of the preceding claims, characterized in that one or more of the elements in the apparatus comprise(s) application or reinforcement parts (3', 19' or 20') of foam with closed alveolae for reinforcing the corrective effect of said elements upon the vertebral column.

10. An apparatus according to any one of claims 1 to 9, characterized in that the means for fixation of the various elements in the apparatus to one another consist of tenons with groove (66) for press-fastener and/or tenons with screws (69, 70), the outer diameter of which corresponds to the inner diameter of the perforations formed in the different elements of the apparatus.

11. An apparatus according to any one of claims 1 to 10, characterized in that it also comprises an occiput and chin support (37) connected to resting points disposed on one or more elements in the apparatus.

12. An apparatus according to claim 11, characterized in that the occiput and chin support (37) comprises two vertical threaded upright rods (38, 39) lying in the patient's symmetrical plane and the upper end of which is respectively connected by means of a ring and a cradle to a chin support (77) and a head rest (78) pivotably mounted and the lower ends of which each rest by means of one or more height adjusting nuts (53) in a telescopic device (42) secured to the pelvian belt.

13. An apparatus according to claim 12, characterized in that each telescopic device (42) comprises a hollow tube (45) receiving the end of a threaded upright rod and provided with a collar (46) and a stroke ring (49) between which at least one spring (51) and a guiding ring (48) are inserted, the tube and both rings being disposed inwardly of a cylinder (43) fast with a fixation system mounted to the pelvian belt.

14. An apparatus according to claim 13, characterized in that each cylinder (43) of the telescopic devices is secured to the belt through a hooking system comprising a pivot for allowing the cylinder to rotate about two horizontal perpendicular axes one of which lies preferably in a removable manner in the patient's symmetrical plane.

15. An apparatus according to claim 13 or 14, characterized in that the ends of the spring (51) of the telescopic device are respectively made fast with the collar (46) and the cylinder (43) and in that the end of the threaded upright rod and that of the tube receiving the latter both comprise means (54, 55) to prevent rotation of the rod in respect to the tube.

FIG.1

FIG.2

FIG.3

FIG.4

FIG.5

FIG.6

FIG.7

FIG.8

1

FIG.12

FIG.13

FIG.9

FIG.14

FIG.15

FIG.16

FIG.17

FIG.18

FIG.19

FIG.20

FIG.21

FIG.22

FIG.23

FIG.10

FIG.11